# EUROPÄISCHE PATENTSCHRIFT

(11) **EP 2 094 329 B1**
(45) Veröffentlichungstag und Bekanntmachung des Hinweises auf die Patenterteilung: **26.02.2014**
(21) Anmeldenummer: 07818581.6
(22) Anmeldetag: 29.09.2007
(51) Int. Cl.: A61M 1/06

(54) **MANUELLE BRUSTPUMPE**
MANUAL BREAST PUMP
TIRE-LAIT MANUEL

(30) Priorität: 24.11.2006 DE 102006056321
(43) Veröffentlichungstag der Anmeldung: 02.09.2009
(73) Patentinhaber: MAPA GmbH, 27404 Zeven (DE)
(72) Erfinder: JÄGER-WALDAU, Reinhold, 27383 Scheessel (DE); MARR, Günter, 21224 Rosengarten (DE)
(74) Vertreter: Siemons, Norbert
(86) Internationale Anmeldenummer: PCT/EP2007/008501
(87) Internationale Veröffentlichungsnummer: WO 2008/061589

(56) Entgegenhaltungen:
- EP-A- 0 116 186
- EP-A- 0 162 358
- EP-A- 1 002 551
- EP-A- 1 184 044
- WO-A-98/29150
- WO-A-2005/016409
- DE-U1- 8 702 791
- FR-A- 1 073 552
- FR-A- 1 104 570
- US-A- 3 977 405
- US-A- 6 110 140

## Beschreibung

Die Erfindung bezieht sich auf eine manuell betreibbare Brustpumpe zum Abpumpen von Mutternilch.

Brustpumpen weisen mindestens eine Saugglocke auf, die an der Mutterbrust angesetzt wird. An die Saugglocke wird ein Unterdruck angelegt, um die Milch aus der Mutterbrust abzuziehen. Die Saugglocke ist mit einem Behälter verbunden, der die abgesaugte Muttermilch aufnimmt..

Aus der EP 0 116 186 A1 ist eine manuell betätigbare Brustpumpe bekannt, die einen länglichen äußeren Zylinder mit einem geschlossenen unteren Ende und einen länglichen rohrförmigen Abschnitt bzw. Hohlkolben aufweist, der am oberen Ende integral mit einer Saugglocke und am unteren Ende lösbar mit einem Dichtring verbunden ist. Der rohrformige Abschnitt ist verschieblich innerhalb des äußeren Zylinders angeordnet, um eine Saugwirkung zu erzielen, wenn die Saugglocke an eine Brust angelegt ist. Der Dichtring hat eine nach oben ragenden Lippe, die zwar beim Ansaugen gut abdichtet, über die jedoch beim Zusammenschieben von rohrförmigem Abschnitt und äußerem Zylinder Milch überströmen kann. Der längliche rohrförmige Abschnitt verjüngt sich, so daß sein Außendurchmesser in der Nähe der Saugglocke kleiner als ein Außendurchmesser in der Nähe seines unteren Endes ist, um ein Kippen der Längsachse des äußeren Zylinders relativ zur Längsachse des rohrförmigen Abschnittes zu ermöglichen, so daß die Saugglocke auch bei einem Kippen des Zylinders ihre Position an der Brust beibehält. Außerdem ist die Saugglocke in einem Winkel zwischen 30° bis 90° bezüglich der Längsachse des länglichen Rohrteiles geneigt, um die Pumpe im Betrieb annähernd vertikal zu halten. Die Saugglocke kann mit einem Adapter bzw. einer Auskleidung versehen werden, um eine Anpassung an eine kleine oder flache Brust vorzunehmen.

Aus der DE 8 702 791 U1 ist eine ähnliche manuelle Brustpumpe mit einem schwenkbar im äußeren Zylinder angeordneten rohrförmigen Abschnitt bekannt, bei der in der Saugglocke ein Einsatzstück aus einem weichen, rückfedernden Polymermaterial angeordnet ist.

Die bekannten manuell betätigbaren Brustpumpen haben den Nachteil, daß der Hohlkolben innerhalb des Zylinders kippen und sich festsetzen kann. Ferner kann es an den Dichtungsringen zum Überströmen von Milch kommen. Aus dem Zylinder in den Hohlkolben rückströmende Milch kann leicht in Kontakt mit der Brust kommen bzw. aus der Saugglocke austreten. Zum Ausführen mehrerer Saughübe muß vor dem Eindrücken des Hohlkolbens in den Zylinder die Saugglocke von der Brust abgenommen werden. Nach dem Eindrücken muß die Saugglocke wieder abdichtend auf der Brust positioniert werden. Wenn der Hohlkolben vollständig in den Zylinder eingeschoben ist, liegt das geneigte Verbindungsrohr zur Saugglocke an dem oberen Rand des Zylinders an. Folglich ist es schwierig, den Hohlkolben festzuhalten, wenn der Zylinder zum Ansaugen nach unten gezogen werden soll.

Der Erfindung liegt die Aufgabe zugrunde, eine manuell betätigbare Brustpumpe mit besseren Anwendungseigenschaften zur Verfügung zu stellen.

Der Erfindung liegt die Aufgabe zugrunde, eine manuell betätigbare Brustpumpe mit besseren Anwendungseigenschaften zur Verfügung zu stellen.

Die Aufgabe wird durch eine Brustpumpe mit den Merkmalen des Anspruchs 1 gelöst.

Gemäß Anspruch 1 hat die manuelle Brustpumpe zum Abpumpen von Muttermilch:
- einen zylindrischen Hohlkolben mit einer umlaufenden Nut am unteren Ende,
- eine Saugglocke, die über einen Fluiddurchgang mit dem oberen Ende des Hohlkolbens verbunden ist,
- eine am unteren Ende des Hohlkolbens angeordnete, umlaufende Doppellippendichtung aus einem weichelastischen Material, die zwei bezüglich eines ringförmigen Grundkörpers in verschiedene Richtungen weisende Dichtlippen aufweist, die jeweils spitzwinklig zur Achse des Hohlkolbens gerichtet sind, wobei der ringförmige Grundkörper auf dem Grund der Nut sitzt, und
- einen Zylinder mit einem geschlossenen unteren Ende, in dem der Hohlkolben axial verschiebbar ist, wobei die Doppellippendichtung abdichtend zwischen der Innenwand des Zylinders und dem Hohlkolben angeordnet ist und zumindest die Saugglocke außerhalb des Zylinders angeordnet ist.

Bei der erfindungsgemäßen Brustpumpe bewirkt die Doppellippendichtung eine effektive Abdichtung des Hohlkolbens bezüglich des Zylinders beim Erzeugen eines Saugunterdruckes und verhindert in besonders effektiver Weise ein Überströmen von Muttermilch aus dem Zylinder in den Zwischenraum zwischen Hohlkolben und Zylinder. Die Dichtigkeit beim Absaugen wird insbesondere durch die vom geschlossenen Ende des Zylinders wegweisende Dichtlippe und die Dichtigkeit gegen das Überströmen von Muttermilch wird insbesondere durch die zum geschlossenen Ende des Zylinders hinweisende Dichtlippe bewirkt.

Die Doppellippendichtung kann verschieden ausgestaltet sein. Sie ist beispielsweise einteilig mit dem Hohlkolben ausgebildet, z.B. durch Anspritzen eines weichelastischen Kunststoffes. Gemäß einer bevorzugten Ausgestaltung ist die Doppellippendichtung eine Doppellippenmanschette, die auf einem Dichtsitz des Hohlkolbens angeordnet ist. Die separat zu montierende Dichtmanschette ermöglicht einen Austausch bei Verschleiß bzw. Beschädigung.

Ferner wird die Aufgabe durch eine Brustpumpe mit den Merkmalen des Anspruches 3 gelöst.

Gemäß einer Ausgestaltung hat die manuelle Brustpumpe zum Abpumpen von Muttermilch:
- einen zylindrischen Hohlkolben,
- eine Saugglocke, die über einen Fluiddurchgang mit dem oberen Ende des Hohlkolbens verbunden ist,
- ein in der Saugglocke angeordnetes Einsatzteil, das randseitig abdichtend am Rand der großen Öffnung der Saugglocke gehalten ist und ein zentrales Durchgangsloch aufweist, wobei zwischen Saugglocke und Einsatzteil ein Stauraum mit mindestens einem weiteren Fluiddurchgang zum Hohlkolben vorhanden ist,
- mindestens ein umlaufendes Dichtelement aus einem weichelastischen Material am unteren Ende des Hohlkolbens und
- einen Zylinder mit einem geschlossenen unteren Ende, in dem der Hohlkolben axial verschiebbar ist, wobei das Dichtelement abdichtend zwischen der Innenwand des Zylinders und dem Hohlkolben angeordnet ist und zumindest die Saugglocke außerhalb des Zylinders angeordnet ist.

Bei der erfindungsgemäßen Brustpumpe tritt zurücklaufende Milch durch den weiteren Fluiddurchgang zwischen der Saugglocke und Einsatzteil in den Stauraum ein und wird darin aufgefangen. Der Kontakt rücklaufender Milch mit der Mutterbrust und die damit verbundenen Unannehmlichkeiten und möglichen Verunreinigungen der Muttermilch sowie ein Auslaufen der Muttermilch aus der Saugglocke werden vermieden. Bei späterer korrekter Ausrichtung der Brustpumpe läuft die Milch in den Zylinder zurück und wird dort aufgefangen.

Das Einsatzteil kann verschieden gestaltet sein. Gemäß einer bevorzugten Ausgestaltung ist es im wesentlichen trichterförmig bzw. konisch. Bei einem trichterförmigen Einsatzteil und einer trichterförmigen bzw. konischen Saugglocke kann der Stauraum dadurch verwirklicht werden, daß das Einsatzteil einen größeren Konuswinkel als die Saugglocke aufweist. Gemäß einer weiteren Ausgestaltung ist das Einsatzteil im wesentlichen trompetenförmig. Durch die trompetenförmige Gestaltung kann bei einer trichterförmigen Saugglocke ein besonders großer Stauraum bereitgestellt werden.

Grundsätzlich kann das Einsatzteil aus einem harten oder hartelastischen Material bestehen. Gemäß einer bevorzugten Ausgestaltung besteht das Einsatzteil aus einem weichelastischen Material, so daß es sich gut an die Brust anpaßt und Lageänderungen des Zylinders durch elastische Verformung kompensiert.

Der weitere Fluiddurchgang kann verschieden ausgestaltet sein. Hierbei kann es sich beispielsweise um mindestens eine Durchgangsöffnung handeln, welche das Einsatzteil abschnittsweise umgibt. Gemäß einer bevorzugten Ausgestaltung ist der weitere Fluiddurchgang ein Ringspalt zwischen der Saugglocke und dem Einsatzteil.

Gemäß einer Ausgestaltung hat die manuelle Brustpumpe zum Abpumpen von Muttermilch:
- einen zylindrischen Hohlkolben,
- eine Saugglocke, die über einen Fluiddurchgang mit dem oberen Ende des Hohlkolbens verbunden ist,
- ein in der Wand der Saugglocke angeordnetes Rückschlagventil, das bei einem Überdruck in der Saugglocke öffnet und ansonsten schließt,
- mindestens ein umlaufendes Dichtelement aus einem weichelastischen Material am unteren Ende des Hohlkolbens und
- einen Zylinder mit einem geschlossenen unteren Ende, in dem der Hohlkolben axial verschiebbar ist, wobei das Dichtelement abdichtend zwischen der Innenwand des Zylinders und dem Hohlkolben angeordnet ist und zumindest die Saugglocke außerhalb des Zylinders angeordnet ist.

Die erfindungsgemäße Brustpumpe braucht bei Durchführung mehrerer Saughübe nicht von der Brust abgesetzt zu werden. Der beim Zusammenschieben von Hohlkolben und Zylinder entstehende Überdruck wird durch das Rückschlagventil abgebaut. Infolgedessen kann ein die Anwendung erschwerendes Abnehmen und wieder Ansetzen der Brustpumpe bei verschiedenen Saughüben bzw. Saughubstellungen entfallen. Vor einem weiteren Saughub ist es nicht erforderlich, eine abdichtende Ansatzposition der Saugglocke zu finden.

Das Rückschlagventil kann verschieden ausgebildet sein, z.B. als Kugelrückschlagventil mit einer verfederten Kugel als Ventilkörper. Gemäß einer Ausgestaltung ist das Rückschlagventil ein Klappenventil. Dies ermöglicht konstruktiv besonders einfache Ausführungen des Rückschlagventils. Gemäß einer weiteren Ausgestaltung weist das Rückschlagventil eine ringförmige Einfassung mit einer am Innenumfang über einen elastischen Steg angelenkten Klappe auf und ist die Einfassung auf einer einen perforierten Boden umgebenden, zylindrischen Sitzfläche der Saugglocke angeordnet, wobei die Klappe im nicht ausgelenkten Zustand an dem perforierten Boden anliegt. Diese Ausgestaltung ist konstruktiv besonders einfach und anwendungsfreundlich. Insbesondere ist ein Austausch des Rückschlagventiles im Falle des Verschleißes oder einer Beschädigung leicht möglich. Gemäß einer weiteren Ausgestaltung ist das Rückschlagventil einteilig aus einem weichelastischen Material hergestellt.

Gemäß einer Ausgestaltung hat die manuelle Brustpumpe zum Abpumpen von Muttermilch:
- einen zylindrischen Hohlkolben mit mindestens einer außen umlaufenden Vertiefung am unteren Ende bezüglich der zylindrischen Mantelfläche des Hohlkolbens,
- eine Saugglocke, die über einen Fluiddurchgang mit dem oberen Ende des Hohlkolbens verbunden ist,
- ein weichelastisches Ausgleichselement zwischen Hohlkolben und Anlagefläche der Saugglocke,
- mindestens ein in die Vertiefung eingesetztes, umlaufendes Dichtelement aus einem weichelastischem Material und
- einen Zylinder mit einem geschlossenen unteren Ende, in dem der Hohlkolben axial verschiebbar ist, wobei die Mantelfläche des Hohlkolbens an einer Innenwand des Zylinders geführt ist, das Dichtelement abdichtend zwischen der Innenwand des Zylinders und dem Hohlkolben angeordnet ist und zumindest die Saugglocke außerhalb des Zylinders angeordnet ist.

Bei der erfindungsgemäßen Brustpumpe wird ein Verkanten und Festsetzen des Hohlkolbens im Zylinder durch die lange Führung des Hohlkolbens an seiner Mantelfläche im Zylinder vermieden. Das Dichtelement sitzt in der Vertiefung innerhalb der Mantelfläche und ragt nur mit einem verformbaren Abschnitt über die Mantelfläche hinaus, der außen abdichtend an der Innenwand des Zylinders anliegt. Aufgrund der Unterbringung des Dichtelementes im wesentlichen innerhalb der Mantelfläche ist es möglich, daß der Innendurchmesser des Zylinders den Außendurchmesser des Hohlkolbens nur geringfügig übersteigt, so daß eine sehr gute Führung des Hohlkolbens im Zylinder erreicht wird. Unterschiedliche Ausrichtungen des Zylinders bei der Anwendung der Brustpumpe werden durch das weichelastische Ausgleichselement kompensiert, so daß die Anlagefläche der Saugglocke ihre Position an der Brust der Benutzerin beibehält. Die mit den verschiedene Ausrichtungen der Brustpumpe zur Anwenderin verbundenen Nachteile werden vermieden.

Das weichelastische Ausgleichselement kann auf verschiedene Weise verwirklicht werden. Beispielsweise ist es ein weichelastisches Rohr oder ein weichelastischer Schlauch zwischen dem oberen Ende des Hohlkolbens und dem Fluiddurchgang der Saugglocke.

Das weichelastische Ausgleichselement ist gemäß einer bevorzugten Ausgestaltung ein in der Saugglocke angeordnetes Einsatzteil aus einem weichelastischen Material, das randseitig abdichtend am Rand der großen Öffnung der Saugglocke gehalten ist und ein zentrales Durchgangsloch aufweist, durch das der Saugunterdruck angelegt wird und die Muttermilch abfließt. Das weichelastische Einsatzteil paßt sich besonders gut an die Mutterbrust an und ist in der Anwendung sehr angenehm. Außerdem behält es bei Lageänderungen der Brustpumpe durch Kippen des Zylinders seine Anlageposition an der Brust bei und gleicht die Lageänderungen durch elastische Verformung aus.

Gemäß einer Ausgestaltung hat die manuelle Brustpumpe zum Abpumpen von Muttermilch:
- einen zylindrischen Hohlkolben,
- eine Saugglocke, die über einen Fluiddurchgang mit dem oberen Ende des Hohlkolbens verbunden ist,
- mindestens ein umlaufendes Dichtelement aus einem weichelastischen Material am unteren Ende des Hohlkolbens und
- einen Zylinder mit einem geschlossenen unteren Ende, in dem der Hohlkolben axial verschiebbar ist, wobei das Dichtelement abdichtend zwischen der Innenwand des Zylinders und dem Hohlkolben angeordnet ist und die Axialerstreckung des Zylinders und des Hohlkolbens so gewählt sind, daß das untere Ende der Saugglocke zumindest eine Daumenbreite über das obere Ende des Zylinders hinausragt, wenn der Hohlkolben maximal in den Zylinder eingeschoben ist.

Bei der erfindungsgemäßen Brustpumpe ist das Greifen des Hohlzylinders durch den zumindest daumenbreiten Überstand der Saugglocke über den Zylinder bei maximal zusammengeschobenem Hohlkolben und Zylinder erleichtert. Die Anwenderin kann zu Beginn des Abpumpvorganges bequem den Hohlkolben zwischen Daumen und Zeigefinger oder anderen einzelnen Fingern festhalten und den Zylinder nach unten ziehen. Eine Daumenbreite entspricht etwa einem Abstand von zwei Zentimetern.

Gemäß einer Ausgestaltung beträgt der Abstand der Saugglocke vom oberen Ende des Zylinders, wenn der Hohlkolben maximal in den Zylinder eingeschoben ist, mindestens zwei Zentimeter. Hierdurch wird das Ergreifen des Hohlkolbens weiter erleichtert.

Unter einer "Saugglocke" wird in dieser Patentanmeldung eine hohle Struktur verstanden, die sich zum einen Ende hin verjüngt und am anderen Ende eine Öffnung aufweist. Die Verjüngung kann viele verschiedene Formen aufweisen, insbesondere trichterförmig bzw. konisch, schalenförmig oder glockenförmig oder anderweitig gewölbt oder gestuft sein, oder verschiedene dieser Formen kombinieren. Die Saugglocke ist bevorzugt so geformt und bemessen, daß sie eine weibliche Brust aufnehmen kann, ohne eine Brustwarze oder den Warzenhof zu berühren.

Grundsätzlich kann die Saugglocke die Form eines Konus aufweisen. Gemäß einer Ausgestaltung weist die Saugglocke eine bezüglich eines Konus ausgebauchte Form auf, in Anpassung an die Form der Brust bzw. zwecks Unterbringung des Stauraums bei der Erfindungsvariante mit einem Stauraum zwischen Saugglocke und Einsatzteil.

Die Saugglocke kann ein separates Teil sein, das über ein Verbindungsteil, z.B. einen Schlauch oder ein Rohr, mit dem Hohlkolben verbunden ist. Gemäß einer Ausgestaltung ist die Saugglocke einteilig mit dem Hohlkolben verbunden.

Nach dem Sammeln der Muttermilch im Zylinder kann damit unterschiedlich verfahren werden. Beispielsweise kann die Muttermilch in eine Saugflasche umgefüllt werden. Der Zylinder kann als Behältnis einer Trinkflasche genutzt werden. Hierfür hat gemäß einer Ausgestaltung der Zylinder am oberen Rand ein Außengewinde. Das Außengewinde ermöglicht das Aufschrauben eines Deckels bzw. eines Trinksaugers.

Gemäß einer weiteren Ausgestaltung hat der Zylinder am unteren Ende einen Standfuß, der die Aufbewahrung des befüllten Zylinders erleichtert. Der Standfuß kann auch zum Greifen des Zylinders beim Saughub genutzt werden. Gemäß einer bevorzugten Ausgestaltung ist der Standruß oval, wodurch die Nutzung des Standfußes als Handgriff begünstigt wird.

Der Zylinder und der Hohlkolben können grundsätzlich aus undurchsichtigem Material gefertigt werden. Gemäß einer Ausgestaltung ist der Zylinder und/oder der Hohlkolben zumindest teilweise transparent. Die Transparenz des Zylinders und/oder des Hohlkolbens erleichtert eine Beobachtung des Abpumpvorganges und der Füllmenge. Gemäß einer weiteren Ausgestaltung ist in einem transparenten Bereich des Zylinders eine Skala mit Volumenangaben vorhanden, die eine Ermittlung der Füllmenge erleichtert.

Für den Hohlkolben und/oder den Zylinder und/oder die Saugglocke kommen verschiedene Materialien in Betracht. Vorzugsweise sind diese Materialien hart oder hartelastisch. Gemäß einer Ausgestaltung sind der Hohlkolben und/oder der Zylinder und/oder die Saugglocke aus PP und/oder PC und/oder PE und/oder ABS und/oder PA und/oder PES und/oder Glas. Insbesondere kann zumindest ein thermoplastischer Kunststoff zum Einsatz kommen. Die genannten Materialien bieten herstellungstechnische Vorteile, sind leicht zu reinigen und hygienisch vorteilhaft.

Gemäß einer Ausgestaltung ist in der Saugglocke ein Einsatzteil angeordnet, das randseitig abdichtend am Rand der großen Öffnung der Saugglocke gehalten ist und ein zentrales Durchgangsloch aufweist.

Grundsätzlich kann die Saugglocke direkt an die Mutterbrust angelegt werden. Das als elastisches Ausgleichselement und als Begrenzung eines Stauraumes bereits erwähnte Einsatzteil kommt gemäß einer weiteren Ausgestaltung bei den weiteren Brustpumpen zum Einsatz, mit den bereits beschriebenen Vorteilen. Gemäß einer Ausgestaltung ist das Einsatzteil trichterförmig oder trompetenförmig, was die dichtende Anlage an die Mutterbrust fördert bzw. die Ausbildung eines Stauraumes zwischen Einsatzteil und Saugglocke unterstützt.

Gemäß einer Ausgestaltung ist das Einsatzteil weichelastisch. Gemäß einer weiteren Ausgestaltung hat das Einsatzteil mehrere Noppen an der Außenseite, die durch den natürlichen Bewegungsablauf und das Hin- und Herbewegen während des Abpumpens eine Massagewirkung entfalten.

Gemäß einer Ausgestaltung ist das Einsatzteil aus Naturkautschuk oder Synthesekautschuk (insbesondere Silikonkautschuk) hergestellt. Ein Einsatzteil aus Kautschuk ist besonders weich, elastisch, leicht zu reinigen und hygienisch.

Gemäß einer konstruktiv besonders einfachen Ausgestaltung ist das Dichtelement ein O-Ring. Es können auch mehrere O-Ringe zum Einsatz kommen. Ferner kann ein O-Ring kombiniert werden mit einer Einfach- oder einer Doppellippendichtung.

Gemäß einer weiteren Ausgestaltung ist das Dichtelement und/oder Rückschlagventil aus Naturkautschuk oder Synthesekautschuk (insbesondere Silikonkautschuk) hergestellt. Ein Dichtelement aus Kautschuk ist ein besonders elastisches, leicht zu reinigendes und hygienisches Material.

Die Erfindung wird nachfolgend anhand der anliegenden Zeichnungen von Ausführungsbeispielen näher erläutert. In den Zeichnungen zeigen:
- Fig. 1: eine manuelle Brustpumpe mit trompetenförmigen Einsatzteilen, Rückschlagventil und Doppellippenmanschette im Längsschnitt;
- Fig. 2: dieselbe Brustpumpe in Längsrichtung geschnitten und in einer Perspektivansicht;
- Fig. 2a: ein Rückschlagventil derselben Brustpumpe in Vorderansicht;
- Fig. 3: dieselbe Brustpumpe in Seitenansicht;
- Fig. 4: Hohlkolben mit Saugglocke derselben Brustpumpe in Seitenansicht;
- Fig. 5: eine Brustpumpe mit transparentem Zylinder in Seitenansicht;
- Fig. 6: dieselbe Brustpumpe in einem Längsschnitt;
- Fig. 7: Brustpumpe mit transparentem Zylinder und verkürztem Einsatzteil im Längsschnitt;
- Fig. 8: Brustpumpe mit transparentem Zylinder und O-Dichtring in einer perspektivischen Seitenansicht;
- Fig. 9: dieselbe Brustpumpe längs geschnitten in derselben Perspektivansicht.

Bei der nachfolgenden Beschreibung verschiedener Ausführungsbeispiele sind dieselben oder im Wesentlichen übereinstimmende Bauteile mit denselben Bezugsziffern versehen.

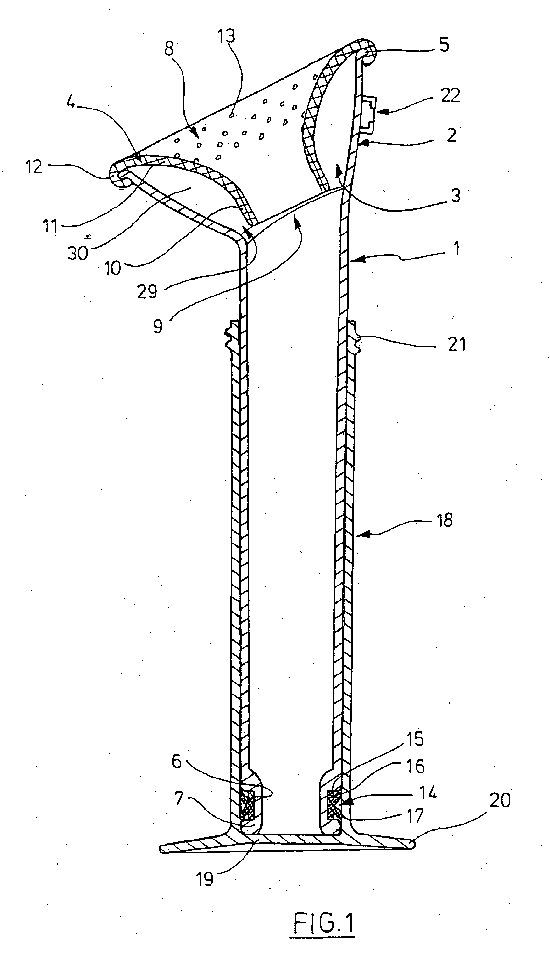

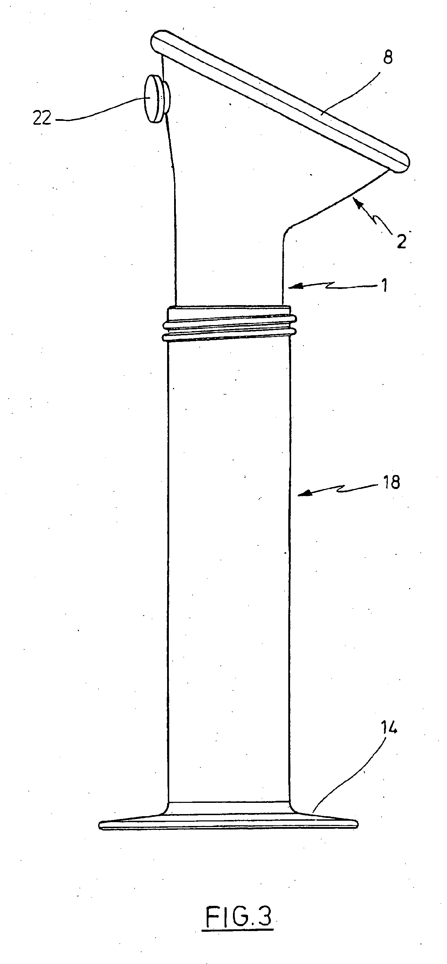

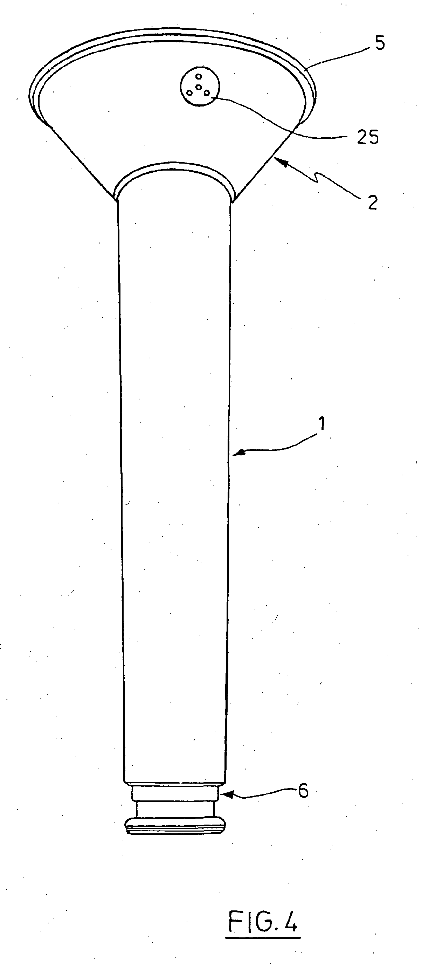

Die Brustpumpe gemäß Fig. 1 bis 4 hat einen zylindrischen Hohlkolben 1, der am oberen Ende einteilig mit einer Saugglocke 2 verbunden ist. Die Saugglocke 2 ist ein hohler Rotationskörper, dessen Mittelachse spitzwinklig zur Mittelachse des Hohlzylinders 1 geneigt ist. Im Verbindungsquerschnitt mit dem Hohlkolben 1 hat die Saugglocke 2 einen Fluiddurchgang 3 zum Hohlkolben 1.

Die Saugglocke 2 hat eine im wesentlichen trichterförmige Form. Ihr Querschnitt erweitert sich allmählich ausgehend von dem Fluiddurchgang 3 bis zu einer großen, kreisförmigen Öffnung 4 am äußeren Ende. Die Öffnung 4 ist von einem Randwulst 5 umgeben. Die Form der Saugglocke 2 ist gegenüber einem Konus etwas nach außen ausgebaucht.

Der Hohlkolben 1 hat am unteren Ende eine Vertiefung in Form einer umlaufenden Nut 6. Angrenzend an das untere Ende hat der Hohlkolben 1 einen Abschnitt vergrößerter Wandstärke 7, der die Nut 6 aufnimmt.

Der Hohlkolben 1 und die Saugglocke 2 sind vorzugsweise integral aus PP oder PC oder Glas hergestellt.

In die Saugglocke 2 ist ein im wesentlichen trichterförmiges Einsatzteil 8 eingesetzt. Die Form des Einsatzteiles 8 kann genauer als trompetenartig beschrieben werden. Das Einsatzteil 8 hat ein zentrales Durchgangsloch 9, an das ein kurzer zylindrischer Abschnitt 10 angrenzt. Vom zylindrischen Abschnitt 10 geht ein allmählich sich nach außen erweiternder Bereich 11 aus. Dieser hat einen zurückgebogenen Rand 12, der über den Randwulst 5 geschnappt und somit an diesem abdichtend fixiert ist.

Auf der Außenseite weist das Einsatzteil 8 eine Vielzahl vorstehender Noppen 13 auf.

Das Einsatzteil 8 ist vorzugsweise integral aus Silikonkautschuk gefertigt.

In der Nut 6 ist eine Doppellippenmanschette 14 angeordnet. Diese hat an einem ringförmigen Grundkörper 15, der auf dem Grund der Nut 6 sitzt, zwei umlaufende Lippen 16, 17, die bezüglich des ringförmigen Grundkörpers 15 in verschiedenen Richtungen spitzwinklig geneigt sind. Die Doppellippenmanschette 14 ist bevorzugt einteilig aus Silikonkautschuk hergestellt.

Der Hohlkolben 1 ist in einen Zylinder 18 eingeschoben, der innen ein geringes Übermaß gegenüber der zylindrischen Mantelfläche des Hohlkolbens 1 aufweist. Das Übermaß ist so gewählt, daß der Hohlkolben 1 leicht innerhalb des Zylinders 18 verschiebbar ist, jedoch so im Zylinder 18 geführt ist, daß er darin nicht verkantet.

Die beiden Lippen 16, 17 der Doppellippenmanschette 14 liegen abdichtend an der Innenwand des Zylinders 18 an.

Der Zylinder 18 ist unten durch einen Boden 19 geschlossen. Der Boden 19 ist Teil eines Standfußes 20, der bezüglich des Zylinders 18 nach außen vorspringt.

Ferner hat der Zylinder 18 am oberen Rand ein Außengewinde 21.

Der Zylinder 18 ist bevorzugt einteilig aus PP, PC oder Glas hergestellt.

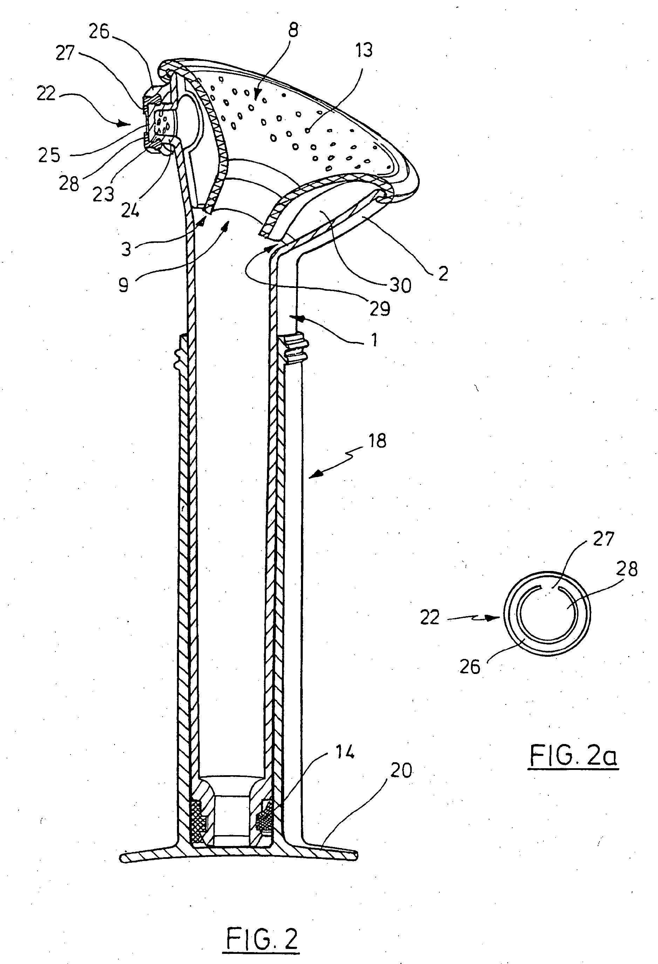

Die Saugglocke 2 trägt ein Rückschlagventil 22. Dieses ist an einer zylindrischen Sitzfläche 23 eines hülsenförmigen Ansatzes 24 positioniert, der auf der Außenfläche der Saugglocke 2 angeordnet ist (vgl. Fig. 2). Der Ansatz 24 hat einen perforierten Boden 25.

Das Rückschlagventil 22 hat eine ringförmige Einfassung 26, die auf der Sitzfläche 23 sitzt. Am inneren Rand der Einfassung ist über einen weichelastischen Steg 27 eine Klappe 28 angebunden, die am perforierten Boden 25 anliegt. Bei Überdruck hebt die Klappe 28 vom perforierten Boden 25 ab.

Gemäß einer anderen Ausgestaltung hat das Ventil an einer Membran einen Ventilkörper. Die Membran ist randseitig außen an der Saugglocke abgestützt und dem Ventilkörper ist ein Dichtsitz in einem Durchgangsloch der Saugglocke zugeordnet.

Wenn in der Saugglocke Unterdruck herrscht oder Normaldruck, sitzt der Ventilkörper auf dem Dichtsitz an und das Durchgangsloch ist geschlossen. Bei Überdruck werden Membran und Ventilkörper ausgelenkt und das Durchgangsloch öffnet sich.

Die Längen des Hohlkolbens 1 und des Zylinders 18 sind so bemessen, daß Saugglocke 2 mindestens eine Daumenbreite über den oberen Rand des Zylinders 18 hinaussteht, wenn der Hohlkolben 1 voll in den Zylinder 18 eingeschoben ist, so daß das untere Ende des Hohlkolbens 1 am Boden 19 anliegt (vgl. Fig. 1 bis 3).

Bei der Anwendung wird das weichelastische Einsatzteil 8 auf die weibliche Brust gesetzt. Die Anwenderin hält den Hohlkolben 1 zwischen Saugglocke 2 und oberem Ende des Zylinders 18 zwischen Daumen und Zeigefinger fest und zieht mit der anderen Hand den Zylinder 18 nach unten. Hierbei strömt Milch in den Zylinder 1 ein. Die Doppellippenmanschette 14 gewährleistet einen hohen Unterdruck beim Saugen und verhindert ein Einströmen von Milch in den Spalt zwischen Hohlkolben 1 und Zylinder 18. Ein Überdruck beim Zurückschieben des Zylinders 18 kann durch das Rückschlagventil 22 entweichen. Falls die Brustpumpe bei rückwärts geneigtem Oberkörper bzw. im Liegen benutzt wird, kann rückströmende Muttermilch allenfalls durch einen Ringspalt 29 in den Stauraum 30 zwischen Einsatzteil 8 und Saugglocke 2 gelangen, so daß ein Kontakt mit der Mutterbrust und ein Auslaufen vermieden werden.

Nach dem Gebrauch kann er Hohlkolben 1 herausgezogen und auf das Außengewinde 21 ein nicht gezeigter Deckel oder ein nicht gezeigter Trinksauger mit Befestigungsring geschraubt werden.

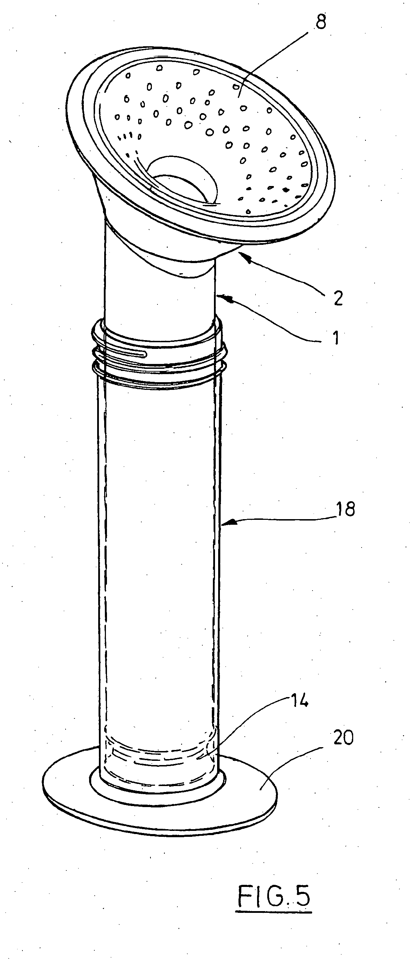

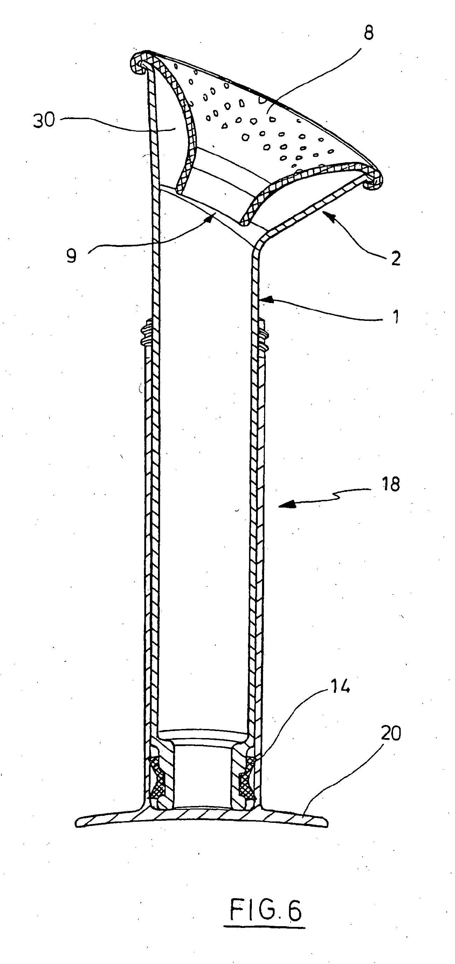

Das Ausführungsbeispiel gemäß Fig. 5 und 6 unterscheidet sich von dem Vorbeschriebenem durch einen transparenten Zylinder 18, der es ermöglicht, die eingesogene Milchmenge zu beobachten. Vorzugsweise ist der Zylinder in senkrechter Richtung mit einer nicht gezeigten Skala versehen. Die Skala weist ggfs. Volumenangaben auf.

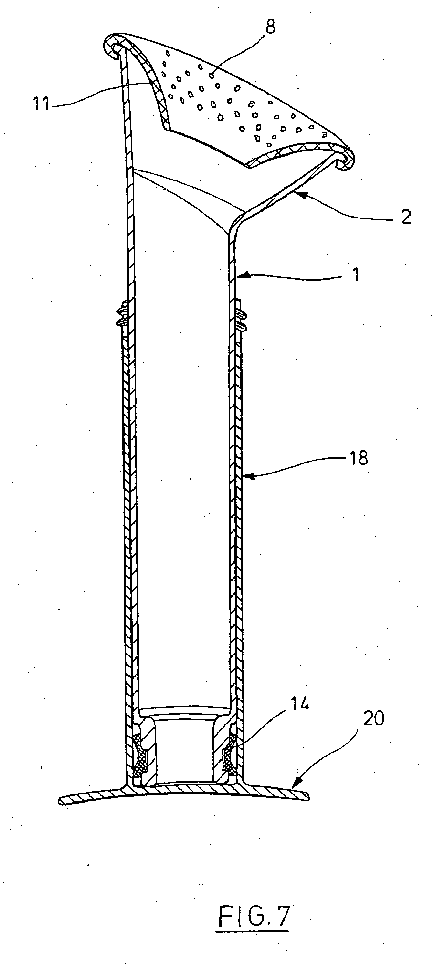

Die Ausführung von Fig. 7 weicht von der Vorstehenden durch die Gestaltung des Einsatzteiles 8 ab. Diesem Einsatzteil 8 fehlt es an dem zylindrischen Abschnitt 10, so daß die kleine Öffnung 9 direkt an den sich erweiternden Abschnitt 11 angrenzt. Diese Ausführung ohne "Kamin" kann bei bestimmten Anwendungen vorteilhaft sein. Außerdem ist kein Rückschlagventil 22 vorhanden.

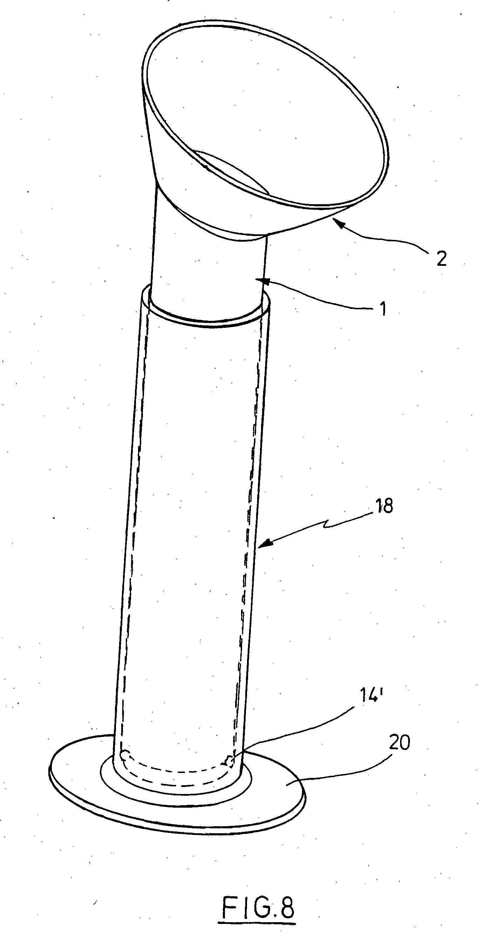

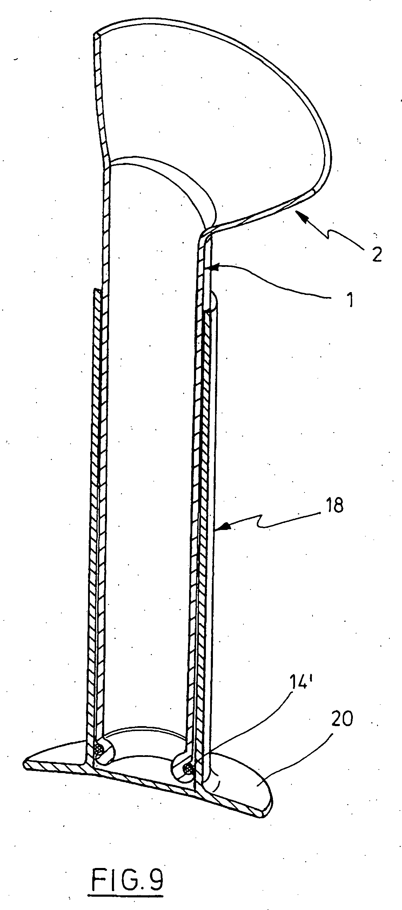

Die Ausführung von Fig. 8 und 9 unterscheidet sich von der gemäß Fig. 5 und 6 dadurch, daß am unteren Ende des Hohlkolbens 1 in einer entsprechend geformten Ringnut 6' ein O-Dichtring 14' angeordnet ist. Außerdem weist die Saugglocke 2 kein Einsatzteil 8 auf und hat der Zylinder 18 kein Außengewinde 21. Bei dieser sehr einfachen Variante steht jedoch die Saugglocke 2 mindestens einen daumenbreit über den Zylinder 18 hinaus.

## Patentansprüche

1. Manuelle Brustpumpe zum Abpumpen von Muttermilch mit
- einem zylindrischen Hohlkolben (1) mit einer umlaufenden Nut (6) am unteren Ende,
- einer Saugglocke (2), die über einen Fluiddurchgang (3) mit dem oberen Ende des Hohlkolbens (1) verbunden ist,
- einer am unteren Ende des Hohlkolbens (1) angeordneten, umlaufenden Doppellippendichtung (14) aus einem weichelastischen Material, die zwei bezüglich eines ringförmigen Grundkörpers (15) in verschiedene Richtungen weisende Doppellippen (16, 17) aufweist, die jeweils spitzwinklig zur Achse des Hohlkolbens (1) gerichtet sind, wobei der ringförmige Grundkörper (15) auf dem Grund der Nut (6) sitzt, und
- einem Zylinder (18) mit einem geschlossenen unteren Ende, in dem der Hohlkolben (1) axial verschiebbar ist, wobei die Doppellippendichtung (14) abdichtend zwischen der Innenwand des Zylinders (18) und dem Hohlkolben (1) angeordnet ist und zumindest die Saugglocke (2) außerhalb des Zylinders (18) angeordnet ist.

2. Brustpumpe nach Anspruch 1, bei der die Doppellippendichtung (14) eine Doppellippenmanschette ist, die auf einem Dichtsitz des Hohlkolbens (1) angeordnet ist.

3. Manuelle Brustpumpe zum Abpumpen von Muttermilch nach einem der Ansprüche 1 bis 2 mit
- einem zylindrischen Hohlkolben (1),
- einer Saugglocke (2), die über einen Fluiddurchgang (3) mit dem oberen Ende des Hohlkolbens (1) verbunden ist,
- einem in der Saugglocke (2) angeordneten Einsatzteil (8), das randseitig am Rand der großen Öffnung (4) der Saugglocke (2) gehalten ist und ein zentrales Durchgangsloch (9) aufweist, wobei zwischen Saugglocke (2) und Einsatzteil (8) ein Stauraum (30) mit mindestens einem weiteren Fluiddurchgang (29) zum Hohlkolben (1) vorhanden ist,
- mindestens einem umlaufenden Dichtelement (14) aus einem weichelastischen Material am unteren Ende des Hohlkolbens (1) und
- einem Zylinder (18) mit einem geschlossenen unteren Ende, in dem der Hohlkolben (1) axial verschiebbar ist, wobei das Dichtelement (14) abdichtend zwischen der Innenwand des Zylinders (18) und dem Hohlkolben (1) angeordnet ist und zumindest die Saugglocke außerhalb des Zylinders (18) angeordnet ist.

4. Brustpumpe nach Anspruch 3, bei der der weitere Fluiddurchgang (29) ein Ringspalt zwischen der Saugglocke (2) und dem Einsatzteil (8) ist.

5. Manuelle Brustpumpe zum Abpumpen von Muttermilch nach einem der Ansprüche 1 bis 4 mit
- einem zylindrischen Hohlkolben (1),
- einer Saugglocke (2), die über einen Fluiddurchgang (3) mit - dem oberen Ende des Hohlkolbens (1) verbunden ist,
- einem in der Wand der Saugglocke (2) angeordneten Rückschlagventil (22), das bei einem Überdruck in der Saugglocke (2) öffnet und ansonsten schließt,
- mindestens einem umlaufenden Dichtelement (14) aus einem weichelastischen Material am unteren Ende des Hohlkolbens (1) und
- einem Zylinder (18) mit einem geschlossenen unteren Ende, in dem der Hohlkolben (1) axial verschiebbar ist, wobei das Dichtelement (14) abdichtend zwischen der Innenwand des Zylinders (18) und dem Hohlkolben (1) angeordnet ist und zumindest die Saugglocke (2) außerhalb des Zylinders (18) angeordnet ist.

6. Brustpumpe nach Anspruch 5, bei der das Rückschlagventil (22) ein Klappenventil ist.

7. Brustpumpe nach Anspruch 6, bei der das Klappenventil (22) eine ringförmige Einfassung (26) mit einer am Innenumfang über einen elastischen Steg (27) angelenkten Klappe (28) aufweist und die Einfassung (26) auf einer einen perforierten Boden (25) umgebenden, zylindrischen Sitzfläche (23) der Saugglocke angeordnet ist, wobei die Klappe (28) im nicht ausgelenkten Zustand an dem perforierten Boden (25) anliegt.

8. Brustpumpe nach Anspruch 7, bei der das Rückschlagventil (22) einteilig aus einem weichelastischen Material hergestellt ist.

9. Brustpumpe zum Abpumpen von Muttermilch nach einem der Ansprüche 1 bis 8 mit
- einem zylindrischen Hohlkolben (1) mit mindestens einer außen umlaufenden Vertiefung (6) am unteren Ende bezüglich der zylindrischen Mantelfläche des Hohlkolbens (1),
- einer Saugglocke (2), die über einen Fluiddurchgang (3) mit dem oberen Ende des Hohlkolbens (1) verbunden ist,
- einem weichelastischen Ausgleichselement (8) zwischen Hohlkolben (1) und Anlagefläche der Saugglocke (2),
- mindestens einem in die Vertiefung (6) eingesetzten, umlaufenden Dichtelement (14) aus einem weichelastischen Material und
- einem Zylinder (18) mit einem geschlossenen unteren Ende, in dem der Hohlkolben (1) axial verschiebbar ist, wobei die Mantelfläche des Hohlkolbens (1) an einer Innenwand des Zylinders (18) geführt ist, das Dichtelement (14) abdichtend zwischen der Innenwand des Zylinders (18) und dem Hohlkolben (1) angeordnet ist und zumindest die Saugglocke (2) außerhalb des Zylinders (18) angeordnet ist.

10. Brustpumpe nach Anspruch 9, bei der das elastische Ausgleichselement ein in der Saugglocke (2) angeordnetes Einsatzteil (8) aus einem weichelastischen Material ist, das randseitig abdichtend am Rand der großen Öffnung (4) der Saugglocke (2) gehalten ist und ein zentrales Durchgangsloch (9) aufweist, durch das der Saugunterdruck angelegt wird und die Muttermilch abfließt.

11. Manuelle Brustpumpe zum Abpumpen von Muttermilch nach einem der Ansprüche 1 bis 10 mit
- einem zylindrischen Hohlkolben (1),
- einer Saugglocke (2), die über einen Fluiddurchgang (3) mit dem oberen Ende des Hohlkolbens (1) verbunden ist,
- mindestens einem umlaufenden Dichtelement (14) aus einem weichelastischen Material am unteren Ende des Hohlkolbens (1) und
- einem Zylinder (18) mit einem geschlossenen unteren Ende, in dem Hohlkolben (1) axial verschiebbar ist, wobei das Dichtelement (14) abdichtend zwischen der Innenwand des Zylinders (18) und dem Hohlkolben (1) angeordnet ist und die Axialerstreckung des Zylinders (18) und des Hohlkolbens (1) so gewählt sind, daß das untere Ende der Saugglocke (2) zumindest eine Daumenbreite über das obere Ende des Zylinders (18) hinausragt, wenn der Hohlkolben (1) maximal in den Zylinder (18) eingeschoben ist.

## Claims

1. A breast pump for pumping down breast milk, with
• a cylindrical hollow piston (1) with at least one groove (6) circulating on the outside, on the lower end,
• a suction cup (2), which is connected to the upper end of the hollow piston (1) via a passage (3) for fluid,
• a double lip seal (14) of soft elastic material on the lower end of the hollow piston (1) which includes double lips (16, 17) with respect to an annular basic body facing into different directions, the lips extending at an acute angle relative to axis of the hollow piston (1), the annular body being positioned at the bottom of the groove (6),
• a cylinder (18) with a closed lower end, in which the hollow piston (1) is axially movable, the double lip seal (14) being sealingly arranged between the inner wall of the cylinder (18) and the hollow piston (1), and at least the suction cup (2) being arranged outside of the cylinder (18).

2. A breast pump according to claim 1, wherein the double lip seal (14) is a double lip sealing member, which is arranged on a sealing seat of the hollow piston (1).

3. A manual breast pump for pumping down breast milk according to claim 1 or 2, with
• a cylindrical hollow piston (1)
• a suction cup (2), which is connected to the upper end of the hollow piston (1) via a passage for fluid (3),
• an insert (8) arranged in the suction cup (2), which is kept at its brim side on the brim of the great opening (4) of the suction cup (2) and which has a central passage hole (9), wherein between suction cup (2) and insert (8) there is a retaining space (30) with at least one further passage for fluid (29) towards the hollow piston (1),
• at least one circulating sealing element (14) made of a soft elastic material on the lower end of the hollow piston (1) and
• a cylinder (18) with a closed lower end, in which the hollow piston (1) is axially movable, wherein the superficies surface of the hollow piston (1) is guided on an inner wall of the cylinder (18), the sealing element (14) is sealingly arranged between the inner wall of the cylinder (18) and the hollow piston (1), and at least the suction cup (2) is arranged outside of the cylinder (18).

4. A breast pump according to claim 3, wherein the further fluid passage (29) is an annular pap between suction cup (2) and insert (8).

5. A manual breast pump for pumping down breast milk according to one of the claims 1 to 4, with
• a cylindrical hollow piston (1);
• a suction cup (2), which is connected to the upper end of the hollow piston (1) via a passage for fluid (3),
• a check valve (22) arranged in the wall of the suction cup (2), which opens at a pressure burden in the suction cup (2) and is otherwise closed,
• at least one circulating sealing element (14) made of a soft elastic material on the lower end of the hollow piston (1), and
• a cylinder (18) with a closed lower end, in which the hollow piston (1) is axially movable, wherein the sealing element (14) is sealingly arranged between the inner wall of the cylinder (18) and the hollow piston (1), and at least the suction cup (2) is arranged outside of the cylinder (18).

6. A breast pump according to claim 5, wherein the check valve (22) is a flap valve.

7. A breast pump according to claim 6, wherein the flap valve (22) has an annular enclosure (26) with a flap (28), articulated on the inside periphery via an elastic bridge (27), and the enclosure is arranged on a cylindrical seating surface (23) of the suction cup (2) surrounding a perforated bottom (25), wherein the flap (28) sits close to the perforated bottom (25) in the not deflected condition.

8. A breast pump according to claim 7, wherein the check valve (22) is made in one piece from a soft elastic material.

9. A manual breast pump for pumping down breast milk according to one of the claims 1 to 8, with
• a cylindrical hollow piston (1) having at least one outer circulating groove (2) at the lower end relative to the cylindrical outer surface of the hollow piston (1),
• a suction cup (2), which is connected to the upper end of the hollow piston (1) via a passage for fluid (3),
• a soft elastic compensation element (8) arranged between the suction cup (2) and the engaging surface of the suction cup (2),
• at least one circulating sealing element (14) made of a soft elastic material on the lower end of the hollow piston (1) and
• a cylinder (18) with a closed lower end in which the hollow piston (1) is axially movable, the outer cylindrical surface of the hollow piston is guided by the inner wall of the cylinder (18), wherein the sealing element (14) is sealingly arranged between the inner wall of the cylinder (18) and the hollow piston (1), and at least the suction cup (2) is arranged outside of the cylinder (18).

10. A manual breast pump according to claim 9, wherein the elastic compensation element (8) arranged in the suction cup (2) is an insert element of elastic material which is kept at its brim side on the brim of the great opening (4) of the suction cup (2) and which has a central passage hole (9) through which mother milk is flowing if suction sub pressure is applied to the central passage hole (9).

11. A manual breast pump for pumping down breast milk according to one of the claims 1 to 10, with
• a cylindrical hollow piston (1),
• a suction cup (2), which is connected to the upper end of the hollow piston (1) via a passage for fluid (3),
• at least one circulating sealing element (14) made of a soft elastic material on the lower end of the hollow piston (1), and
• a cylinder (18) with a closed lower end, in which the hollow piston (1) is axially movable, wherein the sealing element (14) is sealingly arranged between the inner wall of the cylinder (18) and the hollow piston (1), and the axial extensions of the cylinder (18) and the hollow piston (1) are selected such that the lower end of the suction cup (2) projects for at least one thumb width over the upper end of the cylinder (18) when the hollow piston (1) is maximally thrust into the cylinder (18).

## Revendications

1. Tire-lait manuel pour tirer du lait maternel, avec
• un piston creux cylindrique (1) avec une rainure périmétrique (6) sur l'extrémité inférieure,
• une cloche aspirante (2), qui est reliée à l'extrémité supérieure du piston creux (1) à travers un passage de fluide (3),
• un joint à deux lèvres périmétrique (14), arrangé sur l'extrémité inférieure du piston creux (1) et fait d'un matériau élastique mou, qui comporte deux lèvres doubles (16, 17) orientées vers différentes directions par rapport à un corps de base annulaire (15), dont chacune est dirigée en un angle aigu à l'axe du piston creux (1), le corps de base annulaire (15) étant assis sur le fond de la rainure (6), et
• un cylindre (18) avec une extrémité inférieure fermée, dans lequel le piston creux (1) est axialement mobile, le joint à deux lèvres (14) étant arrangé de manière étanche entre la paroi intérieure du cylindre (18) et le piston creux (1), et au moins la cloche aspirante (2) étant arrangée au-dehors du cylindre (18).

2. Tire-lait selon la revendication 1, dans lequel le joint à deux lèvres (14) est un manchon à deux lèvres qui est arrangé sur un siège d'étanchéité du piston creux (1).

3. Tire-lait manuel pour tirer du lait maternel selon l'une quelconque des revendications 1 à 2, avec
• un piston creux cylindrique (1),
• une cloche aspirante (2), qui est reliée à l'extrémité supérieure du piston creux (1) à travers un passage de fluide (3),
• une pièce d'insertion (8) arrangée dans la cloche aspirante (8), qui est tenue dans son côté bord sur le bord de la grande ouverture (4) de la cloche aspirante (2) et qui comporte un trou traversant (9) central, une capacité de retenue (30) avec au moins un autre passage de fluide (29) vers le piston creux (1) existant entre la cloche aspirante (2) et la pièce d'insertion (8),
• au moins un élément d'étanchéité périmétrique (14) fait d'un matériau élastique mou sur l'extrémité inférieure du piston creux (1), et
• un cylindre (18) avec une extrémité inférieure fermée, dans lequel le piston creux (1) est axialement mobile, l'élément d'étanchéité (14) étant arrangé de manière étanche entre la paroi intérieure du cylindre (18) et le piston creux (1), au moins la cloche aspirante étant arrangée au-dehors du cylindre (18).

4. Tire-lait selon la revendication 3, dans lequel l'autre passage de fluide (29) est une fente annulaire entre la cloche aspirante (2) et la pièce d'insertion (8).

5. Tire-lait manuel pour tirer du lait maternel selon l'une quelconque des revendications 1 à 4, avec
• un piston creux cylindrique (1),
• une cloche aspirante (2), qui est reliée à l'extrémité supérieure du piston creux (1) à travers un passage de fluide (3),
• une soupape de retenue (22), arrangée dans la paroi de la cloche aspirante (8), qui s'ouvre dans une surpression dans la cloche aspirante (2), et est autrement fermée,
• au moins un élément d'étanchéité périmétrique (14) fait d'un matériau élastique mou sur l'extrémité inférieure du piston creux (1), et
• un cylindre (18) avec une extrémité inférieure fermée, dans lequel le piston creux (1) est axialement mobile, l'élément d'étanchéité (14) étant arrangé de manière étanche entre la paroi intérieure du cylindre (18) et le piston creux (1), au moins la cloche aspirante (2) étant arrangée au-dehors du cylindre (18).

6. Tire-lait selon la revendication 5, dans lequel la soupape de retenue (22) est une soupape à clapet.

7. Tire-lait selon la revendication 6, dans lequel la soupape à clapet (22) comporte un cadre annulaire (26) avec un clapet (28) articulé sur le périmètre intérieur au moyen d'une membrure élastique (27), et le cadre (26) est arrangé sur une surface d'appui cylindrique (23) de la cloche aspirante entourant un fond perforé (25), le clapet (28) étant en contact avec le fond perforé (25) dans la condition non déplacée.

8. Tire-lait selon la revendication 7, dans lequel la soupape de retenue (22) est faite comme une seule pièce en un matériau élastique mou.

9. Tire-lait pour tirer du lait maternel selon l'une quelconque des revendications 1 à 8, avec
• un piston creux cylindrique (1) avec au moins une concavité (6) sur le périmètre extérieur dans l'extrémité inférieure par rapport à la surface d'enveloppe cylindrique du piston creux (1),
• une cloche aspirante (2), qui est reliée à l'extrémité supérieure du piston creux (1) à travers un passage de fluide (3),
• un élément de compensation (8) élastique mou entre le cylindre creux (1) et la surface d'appui de la cloche aspirante (2),
• au moins un élément d'étanchéité périmétrique (14) fait d'un matériau élastique mou et inséré dans la concavité (6), et
• un cylindre (18) avec une extrémité inférieure fermée, dans lequel le piston creux (1) est axialement mobile, la surface d'enveloppe du piston creux (1) étant guidée sur une paroi intérieure du cylindre (18), l'élément d'étanchéité (14) étant arrangé de manière étanche entre la paroi intérieure du cylindre (18) et le piston creux (1), au moins la cloche aspirante étant arrangée au-dehors du cylindre (18).

10. Tire-lait selon la revendication 9, dans lequel élément de compensation élastique est une pièce d'insertion (8) en un matériau élastique mou arrangée dans la cloche aspirante (8), qui est tenue de manière étanche dans son côté bord sur le bord de la grande ouverture (4) de la cloche aspirante (2) et qui comporte un trou traversant (9) central à travers la dépressurisation aspirante est appliquée et le lait maternel s'écoule.

11. Tire-lait manuel pour tirer du lait maternel selon l'une quelconque des revendications 1 à 10, avec
• un piston creux cylindrique (1),
• une cloche aspirante (2), qui est reliée à l'extrémité supérieure du piston creux (1) à travers un passage de fluide (3),
• au moins un élément d'étanchéité périmétrique (14) fait d'un matériau élastique mou sur l'extrémité inférieure du piston creux (1), et
• un cylindre (18) avec une extrémité inférieure fermée, dans lequel le piston creux (1) est axialement mobile, l'élément d'étanchéité (14) étant arrangé de manière étanche entre la paroi intérieure du cylindre (18) et le piston creux (1) et l'extension axiale du cylindre (18) et du piston creux (1) étant choisie tellement que l'extrémité inférieure de la cloche aspirante (2) fait saillie pour au moins un pouce au-delà de l'extrémité supérieure du cylindre (18) quand le piston creux (1) est maximalement inséré dans le cylindre (18).
